# DEMANDE DE BREVET EUROPEEN

(11) **EP 4 616 863 A1**
(43) Date de publication de la demande: **17.09.2025**
(21) Numéro de dépôt: 24305370.9
(22) Date de dépôt: 12.03.2024
(51) Int. Cl.: A61K 35/56, A61K 39/395, A61P 31/14, A61K 39/00, A61K 35/64, A61K 39/39

(54) **VACCIN CONTRE LES VESICULES EXTRACELLULAIRES DE MOUSTIQUE POUR PROTEGER CONTRE LES FLAVIVIRUS**

(71) Demandeur: Institut de Recherche pour le Développement, 13572 Marseille Cedex 02 (FR); Centre National de la Recherche Scientifique, 75016 Paris (FR); UNIVERSITE DE MONTPELLIER, 34090 Montpellier (FR)
(72) Inventeur: POMPON, Julien Francis, 34170 Castelnau le lez (FR); MEDKOUR, Hacène, 34000 Montpellier (FR); MIOT, Elliott, 34090 Montpellier (FR)
(74) Mandataire: Novagraaf Technologies

(57) **Abrégé**

La présente invention se rapporte à une vésicule extracellulaire isolée de moustique pour son utilisation comme agent immunogène dans la prévention d'au moins une maladie à transmission vectorielle.

La présente invention se rapporte également à une composition vaccinale comprenant au moins une vésicule extracellulaire de moustique.

La présente invention se rapporte en outre à une utilisation d'une vésicule extracellulaire de moustique pour identifier au moins un peptide immunogène capables de réduire, chez un vertébré la transmission d'une maladie à transmission vectorielle lors d'une piqûre du vertébré par le vecteur, ainsi qu'à un procédé d'identification d'au moins un peptide immunogène capable de bloquer, chez un vertébré, la transmission d'une maladie à transmission vectorielle lors d'une piqûre du vertébré par le vecteur, comprenant une étape d'identification *in vitro* d'au moins une cible d'anticorps présents dans un échantillon de sérum d'animal immunisé contre des vésicules extracellulaires de moustique.

## Description

### Domaine technique

La présente invention se rapporte à un produit pour son utilisation comme agent immunogène dans la prévention d'au moins une maladie à transmission vectorielle, ainsi qu'à une composition vaccinale comprenant ce produit, et à l'utilisation de ce produit pour identifier au moins un peptide immunogène capables de réduire, chez un vertébré, la transmission d'une maladie à transmission vectorielle lors d'une piqûre du vertébré par le vecteur.

La présente invention trouve une application dans le domaine pharmaceutique, notamment dans le domaine des vaccins.

### Etat de la technique

Les flavivirus transmis par les moustiques incluent les virus de la dengue, du Zika, de la fièvre jaune, de l'encéphalite japonaise et de la fièvre du Nil Occidental. A eux seuls, ces flavivirus sont responsables de près d'un demi-milliard d'infections par an, causant la mort d'environ 250 000 personnes et coutant plus de 10 milliards d'euros. De plus, du fait de la répartition géographique des moustiques vecteurs, la quasi-totalité de l'humanité vit dans une zone à risque d'infection. Malgré cette situation alarmante, il n'existe pas de moyens de lutte efficace : il n'existe pas de traitement curatif, la lutte antivectorielle a une efficacité modérée et la stratégie vaccinale est dans une impasse car les vaccins actuels ciblant les flavivirus augmentent la sévérité des symptômes dans certaines conditions.

En effet, il existe actuellement plusieurs limites aux stratégies vaccinales contre les flavivirus. D'une part, il existe une demande croissante de vaccins contre plusieurs flavivirus pour répondre à la diversité grandissante du risque viral. En effet, plusieurs flavivirus menacent actuellement les populations humaines et l'émergence de nouveaux flavivirus comme pathogènes importants est attendue dans un futur proche du fait des changements globaux. Cependant, il n'existe que quelques vaccins contre les flavivirus qui ciblent la fièvre jaune, l'encéphalite Japonaise et la dengue. Ainsi, l'humanité va être confrontée à une multiplication des besoins en vaccin contre un nombre croissant de flavivirus. D'autre part, les vaccins ciblant les protéines flavivirales peuvent comporter des risques sanitaires. Le récent exemple du vaccin contre la dengue, DENGVAXIA^{®} (SANOFI-Pasteur) en est une illustration. Ce vaccin déployé aux Philippines a révélé que la vaccination accroît le risque de formes graves de la dengue, dans certaines conditions. En effet, les enfants vaccinés contre la dengue avaient un risque accru de mortalité lorsqu'ils n'avaient jamais été infectés par le virus avant l'immunisation. Ce phénomène est lié à l'amplification de l'infection par les anticorps non-neutralisants générés lors de la vaccination. Ces anticorps facilitent l'internalisation du virus dans les cellules de type macrophage, augmentant l'infection et la gravité des symptômes. De façon encore plus alarmante, cette amplification de l'infection par les anticorps non-neutralisants pourrait aussi fonctionner entre espèce de flavivirus car les anticorps contre une première infection de flavivirus augmentent l'infection secondaire par une autre espèce de flavivirus. Ces données suggèrent fortement qu'une réponse immunitaire induite par un vaccin contre une espèce de flavivirus pourrait aggraver l'infection par une autre espèce de flavivirus et questionne sérieusement la stratégie vaccinale qui consiste à cibler les protéines flavivirales.

Il existe donc un réel besoin d'un nouvel outil de prévention contre les flavivirus, palliant ces défauts, inconvénients et obstacles de l'art antérieur.

### Description de l'invention

Aux termes d'importantes recherches, les inventeurs ont mis au point un vaccin à large-spectre contre plusieurs flavivirus, en ciblant les vésicules extracellulaires de moustique.

Les inventeurs de la présente ont mis en évidence de manière surprenante que les vésicules extracellulaires (VE) de la salive de moustique favorisent l'infection de plusieurs flavivirus.

En effet, les inventeurs ont montré de manière inattendue que les lipides contenus dans les VE de moustique amplifient l'infection de la peau et la transmission virale. Ils ont de plus démontré que la supplémentation en lipides de VE augmente l'infection par plusieurs flavivirus sur plusieurs modèles cellulaires pertinents pour la transmission, notamment des cellules primaires de fibroblastes cutanées et des cellules immunitaires primaires infectées prioritairement par les virus lors de la piqûre. Les inventeurs ont en outre validé l'effet des lipides des VE avec un modèle murin en montrant que l'injection du virus supplementée par des lipides des VE de moustique augmente l'infection et les symptômes sur un modèle animal. Ainsi, les inventeurs ont identifié un facteur d'amplification de la transmission pan-flavivirale.

En se basant sur ces informations, les inventeurs ont mis au point une nouvelle stratégie vaccinale qui consiste à immuniser contre ces vésicules extracellulaire de moustique pour altérer l'initiation de l'infection de la peau et donc réduire la pathogénèse virale et/ou les symptômes de l'infection.

Les inventeurs de la présente sont ainsi les tout premiers à avoir mis en évidence, de manière tout à fait inattendue, qu'une immunisation avec des vésicules extracellulaires de moustique protège contre l'infection par des flavivirus.

Les inventeurs ont notamment démontré que l'immunisation de souris avec des vésicules de moustique protège contre l'infection par le virus du Nil Occidental par piqûre de moustique.

Ainsi, l'invention apporte une solution technique présentant les avantages suivants :
- Une protection pan-flavivirale. En effet, en ciblant un facteur nécessaire à l'infection de plusieurs flavivirus, l'invention protège contre plusieurs flavivirus avec un seul vaccin.
- Une sécurité sanitaire accrue. En ciblant des protéines salivaires du moustique, l'invention contourne les risques sanitaires associés à la stratégie vaccinale ciblant les protéines flavivirales.

Ainsi, un premier objet de l'invention se rapporte à une vésicule extracellulaire isolée de moustique pour son utilisation comme agent immunogène dans la prévention d'au moins une maladie à transmission vectorielle.

On entend par « vésicule extracellulaire », également désignée sous l'acronyme « VE », au sens de la présente invention, toute vésicule non-réplicative sécrétée par une cellule de moustique, notamment par les cellules des glandes salivaires. Les VE sont délimitées par une bicouche lipidique maintenue par des protéines transmembranaires, qui ont une partie dans le lumen et une partie externe à la VE. Les VE peuvent être internalisées par des cellules réceptrices et ainsi transférer leur matériel de la cellule sécrétrice à une cellule réceptrice.

Les vésicules extracellulaires peuvent être isolées à partir d'au moins un liquide biologique sécrété par un moustique, comme par exemple la salive, l'hémolymphe ou les excrétats. Alternativement, les vésicules extracellulaires peuvent être isolées à partir d'au moins un extrait cellulaire de moustique, comme par exemple une culture de cellules, un extrait de glandes salivaire, un broyat d'oeufs, un broyat d'intestin, un broyat de larves, un broyat du corps du moustique ou une culture *ex vivo* de tissu ou d'organe de moustique. Dans le cas d'une culture de cellules, les cellules peuvent être choisies parmi les lignées cellulaires *Aedes aegypti* Aag2, RML-12 et CCL125, *Aedes albopictus* C6/36, C7-10 et U4.4, *Culex quinquefasciatus* Hsu, *Culex tritaeniorhynchus* TRA-171 et *Anopheles gambiae* Mos.55, Sua1B ou 4a-3B. Avantageusement, ces modèles cellulaires permettent de s'affranchir des quantités limitées de salive produite par les moustiques.

Les liquides biologiques ou les extraits cellulaires précités peuvent être issus de moustique infecté ou de cellules infectées, ou alternativement de moustique ou de cellules non infecté(es). En d'autres termes, les VE peuvent contenir des virus dans le cas où elles sont issues de moustique, de liquide, d'extrait ou de cellules infecté(es), ou ne pas en contenir dans le cas contraire. Avantageusement, l'utilisation de liquide biologique, d'extrait ou de cellules non-infecté(es) permet de ne pas introduire de matériel viral lors de l'immunisation et/ou de ne pas avoir à isoler les VE des virus avant l'immunisation. En effet, la présence de protéine virale déclencherait une immunisation contre ces protéines virales, outre l'immunisation crée par les VE et cette immunisation contre les protéines virales protégèrerait contre l'infection, masquant ainsi l'effet protecteur des VE.

Les vésicules extracellulaires peuvent être isolées par tout procédé de purification connu de l'homme du métier, par exemple par ultracentrifugation différentielle, séparation par gradient de densité, filtration par taille, immuno-affinité, affinité pour certains lipides, affinité par les charges de surface, chromatographie d'exclusion de taille, kits de précipitation, filtres centrifuges à seuil de poids moléculaires, filtration par flux tangentiel, colone d'affinité pour membrane, cytométrie à flux, cette liste n'étant pas limitative.

Le moustique peut être toute espèce de moustique vecteur de virus, notamment de flavivirus. Il peut s'agir par exemple d'un moustique du genre Aedes, par exemple *Aedes aegypti, Aedes albopictus* ou *Aedes japonicus*, ou du genre *Culex,* par exemple *Culex pipiens*, *Culex quinquefasciatus, Culex tarsalis, Culex molestus, Culex tritaeniorhynchus,* ou encore du genre *Anopheles*, comme *Anopheles gambiae*, *Anopheles coluzii*, ou *Anopheles arabiensis.*

La maladie à transmission vectorielle peut être par exemple au moins une maladie choisie parmi la dengue, le Zika, la fièvre jaune, l'encéphalite japonaise, la fièvre du Nil Occidental, l'encéphalite de Saint Louis, la maladie causée par le virus Tembusu et la maladie causée par le virus Usutu.

Les vésicules extracellulaires sont utilisées comme « agent immunogène », au sens de la présente invention, pour leur capacité à déclencher une réponse immunitaire dans un organisme. Cette réponse immunitaire est dirigée contre tout ou partie des vésicules extracellulaires.

On entend par « prévention », au sens de la présente invention, la diminution du risque de contracter une maladie ou le fait d'en atténuer les effets, dans un organisme, par rapport à un organisme dans lequel l'acte préventif, à savoir l'administration de VE, n'a pas eu lieu. Avantageusement, la prévention est la conséquence d'une réponse immunitaire dans l'organisme suite à l'administration des VE. Sans vouloir être lié par une théorie ou un mécanisme d'action, l'administration des VE déclencherait la production d'anticorps et activerait les cellules immunitaires spécifiques capables de reconnaître les VE et d'empêcher l'infection de la peau, nécessaire à l'infection systémique et à la transmission. En effet, l'étape d'initiation de l'infection de la peau est un point faible de la transmission car seulement quelques centaines de virus infectieux sont injectés avec la salive de moustique. De plus, bloquer l'infection de la peau empêche la transmission car l'infection de la peau est nécessaire à l'infection systémique et la transmission. En conséquence, la prévention selon l'invention permettrait de réduire ou d'empêcher la transmission de la maladie lors d'une piqûre.

La prévention peut avoir lieu par administration de VE isolée, chez un vertébré humain ou non humain. Parmi les vertébrés non humains, on peut citer par exemple les mammifères, comme les singes, les chats, les chiens, les vaches, les moutons, les lapins, les cochons, les chèvres, les chevaux, ou les ânes, les oiseaux, comme les canards, les dindes ou les poulets, ou les poissons, cette liste n'étant pas limitative.

Un autre objet de l'invention se rapporte à une composition vaccinale comprenant au moins une vésicule extracellulaire de moustique telle que définie ci-avant. La composition vaccinale possède les effets de prévention d'au moins une maladie à transmission vectorielle tels que décrits ci-avant.

La composition vaccinale de l'invention peut comprendre en outre tout ingrédient supplémentaire classiquement utilisé dans ce type de formulation spécifique, notamment pour assurer son efficacité et sa sécurité en fonction du vertébré à vacciner. Il peut s'agir d'au moins une substance choisie parmi les véhicules, les excipients, les adjuvants, les tampons, les conservateurs, les régulateurs immunitaires et les stabilisants. Il peut s'agir par exemple de sucres, comme le saccharose, le sorbitol, de protéines, comme l'albumine humaine ou le sérum bovin, de polyols, comme la glycérine, des acides aminés, du phénol, du phénoxyéthanol, d'agonistes immunitaire, de sels, comme les sels d'aluminium, les phoshates d'aluminium, d'émulsions, comme les émulsions à base de squalènes, ou de liposomes, cette liste n'étant pas limitative.

La composition peut être sous toute forme classiquement utilisée dans le domaine des vaccins. Il peut s'agir par exemple d'une forme choisie parmi la fome injectable, la forme orale, la forme intranasale, la forme intradermique, la forme transdermique, la forme intramusculaire ou la voie oropharyngée. De préférence, il s'agit d'une forme injectable.

Selon l'invention, la composition peut comprendre toute dose pharmaceutiquement acceptable et efficace de vésicule extracellulaire. La dose peut être adaptée au vertébré à vacciner par l'homme du métier, en fonction de ses connaissances générales. Il peut s'agir d'une dose permettant l'administration de VE entre 35 et 100 nmol d'équivalent protéines chez l'homme, ou entre 5 et 20 nmol chez la souris, ou entre 20 et 50 nmol chez le lapin.

Un autre objet de l'invention se rapporte à un procédé de préparation de vésicule extracellulaire de moustique, comprenant les étapes suivantes :
1) culture de cellules de moustique, non infectées ou infectées par un virus,
2) séparation du surnageant de culture,
3) purification des vésicules extracellulaires du surnageant de culture, par exemple par ultracentrifugation.

Les cellules de moustique sont telles que définies ci-avant. De préférence, il peut s'agir de la lignée cellulaire *Aedes aegypti* Aag2.

Les conditions *in vitro* et le milieu de culture utilisés sont ceux connus de l'homme du métier, notamment en fonction de la lignée cellulaire choisie, pour permettre aux cellules de se diviser et de sécréter les vésicules extracellulaires.

L'étape de séparation du surnageant de culture, qui contient les vésicules extracellulaires, peut être réalisée par toute technique connue de l'homme du métier, comme par exemple la centrifugation, la filtration, la sédimentation, la chromatographie ou l'ultrafiltration.

L'étape de séparation des vésicules extracellulaires du surnageant de culture peut être réalisée par toute technique connue de l'homme du métier, comme par exemple par ultracentrifugation différentielle, séparation par gradient de densité, filtration par taille, immuno-affinité, affinité pour certains lipides, affinité par les charges de surface, chromatographie d'exclusion de taille, kits de précipitation, filtres centrifuges à seuil de poids moléculaires, filtration par flux tangentiel, colone d'affinité pour membrane, cytométrie à flux, cette liste n'étant pas limitative. De préférence, il peut s'agir d'une ultracentrifugation.

Les vésicules extracellulaires obtenues sont notamment des vésicules intactes, ou non intactes permettant l'immunisation d'un vertébré. On entend par « vésicule intacte » une vésicule dont la membrane bicouche lipidique n'est pas altérée. Ceci permet aux VE de conserver un contenu à l'intérieur de la membrane bicouche lipidique et de présenter les antigènes en surface. On entend par « vésicule non intacte » une vésicule dont la membrane en bicouche lipidique est altérée. Il peut s'agir par exemple d'un fragment de vésicule, ou d'une vésicule dont la membrane est dénaturée, ou interrompue à un ou plusieurs endroits.

Un autre objet de l'invention se rapporte à l'utilisation d'une vésicule extracellulaire de moustique pour identifier, *in vitro,* au moins un peptide immunogène capables de réduire, chez un vertébré la transmission d'une maladie à transmission vectorielle lors d'une piqûre du vertébré par le vecteur.

Un autre objet de l'invention se rapporte à un procédé d'identification d'au moins un peptide immunogène capable de bloquer ou réduire, chez un vertébré, la transmission d'une maladie à transmission vectorielle lors d'une piqûre du vertébré par le vecteur, comprenant une étape d'identification *in vitro* d'au moins une cible d'anticorps présents dans un échantillon de sérum d'animal immunisé contre des vésicules extracellulaires de moustique.

Avantageusement, les peptides immunogènes identifiés peuvent correspondre à tout ou partie de la partie externe, ou à tout ou partie de la partie interne, ou à tout ou partie de la partie transmembranaire, d'une protéine transmembranaire de la vésicule extracellulaire de moustique.

L'étape d'identification des cibles des anticorps présents dans l'échantillon de sérum peut être réalisée par toute technique connue de l'homme du métier. Il peut s'agir par exemple de l'Enzyme-Linked Immunosorbent Assay (ELISA), l'immunoprécipitation, le Western blot, l'immunofluorescence, un criblage par affichage sur levure, l'immunohistochimie ou les puces à protéines.

D'autres avantages pourront encore apparaître à l'homme du métier à la lecture des exemples ci-dessous, illustrés par les figures annexées, donnés à titre illustratif.

### Brève description des figures

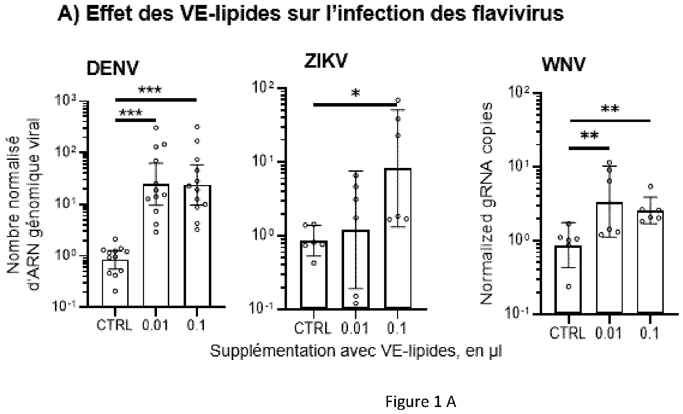

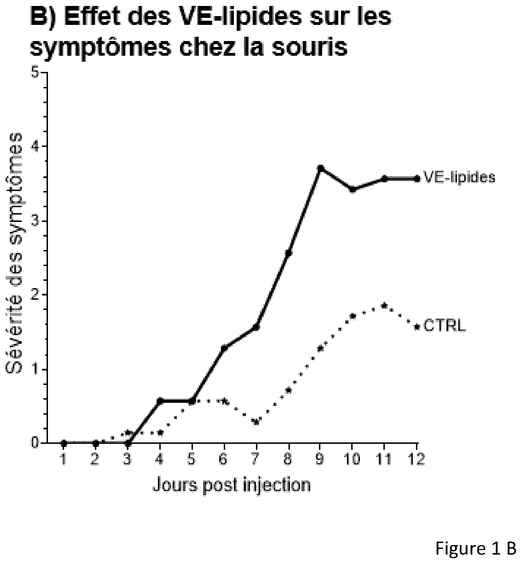

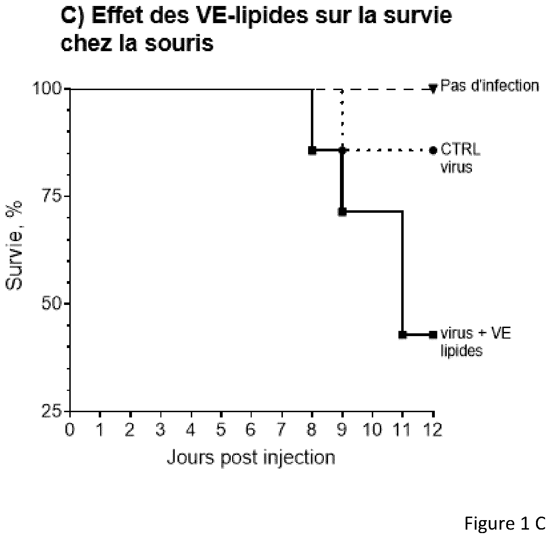

- La figure 1 représente : A) le nombre normalisé de copies d'ARN génomique du virus dans des cellules humaines de foie (Huh7) infectées par le virus de la Dengue (DENV), le virus Zika (ZIKV) et le virus de la fièvre du Nil Occidental (WNV), supplémentées avec des lipides issus de vésicules extracellulaires (VE-lipides ; volume = 0.01 ou 0.1 µl d'extrait de lipides) ou dans le groupe contrôle sans supplémentation (CTRL). * indique une valeur du p statistique < 0.05 ; ** < 0.01 ; et *** < 0.001 ; selon un test T. B) La sévérité des symptômes chez une souris infectée par injection du virus de la fièvre du Nil Occidental (WNV) supplémentée par des lipides de VE de moustique (VE-lipides) ou non (CTRL), en fonction du nombre de jours post-injection. C) Survie des souris infectées par injection du virus de la fièvre du Nil Occidental (WNV) supplémentée par des lipides de VE (virus + VE lipides), ou non (CTRL virus), ou sans infection, en fonction du nombre de jours post-injection.

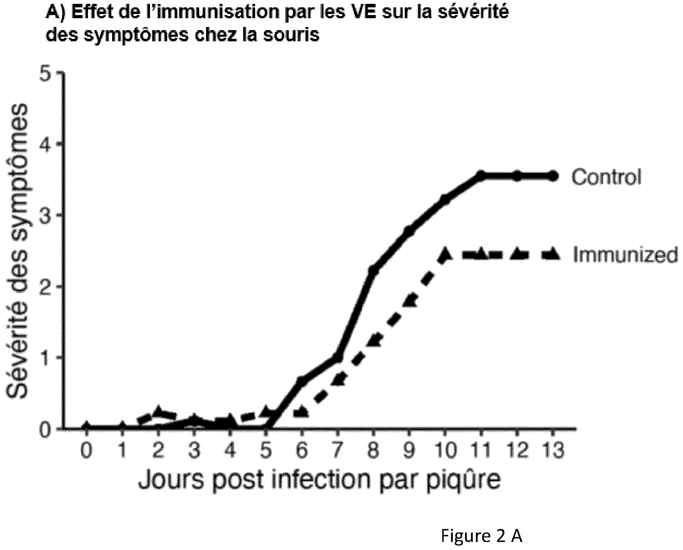

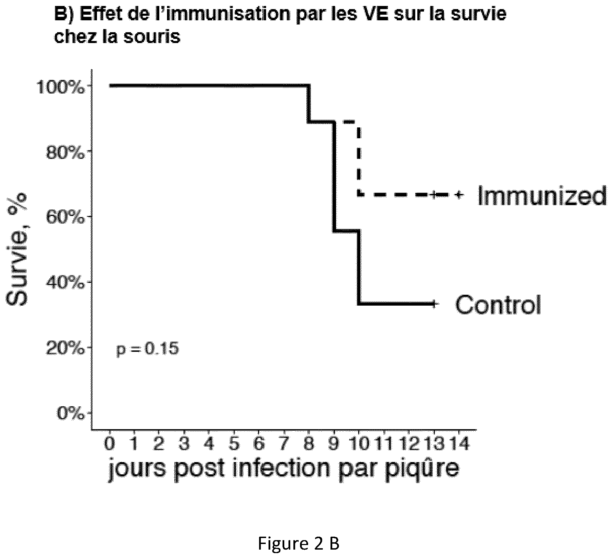

- La figure 2 représente A) La sévérité des symptômes après piqûre de moustique infectés par le virus du Nil Occidental pour des souris contrôles non-immunisées (CTRL) ou immunisées avec des VE de moustique (Immunisée) en fonction du nombre de jours après l'infection par piqûre. B) Le pourcentage de survie pour le groupe contrôle (CTRL) et pour le groupe immunisé par des VE de moustique (immunisé) en fonction du nombre de jours après l'infection par piqûre.

### EXEMPLES

### Exemple 1 : préparation de vésicules extracellulaires isolées

Les VE sont purifiées à partir de la lignée cellulaire Aag2 (Aedes aegypti) (Peleg, J. "Growth of Arboviruses in Primary Tissue Culture of Aedes Aegypti Embryos." American Journal of Tropical Medicine and Hygiene 17, no. 2 (1968): 219-, ([1])).

Il est à noter que les cellules Aag2 utilisées pour la purification des VE ne sont pas infectées par un virus, notamment pas par un flavivirus. L'utilisation de cellules non-infectées permet de ne pas introduire de matériel viral lors de l'immunisation. En effet, la présence de protéines virales aurait déclenché une immunisation contre ces protéines virales et l'effet des VE n'aurait pas pu être évalué en tant que tel.

### Matériel

- cellules Aag2
- milieu Roswell Park Memorial Institute (RPMI) medium 1640, GlutaMAX^{™} supplément
- Penicillin-Streptomycin (PS) (10,000 U/mL)
- Non-Essential Amino Acids (NEAA) Solution (100X)
- Sérum de veau foetal (FCS)
- solution saline tamponnée au phosphate de Dulbecco (DPBS), dépourvue de calcium et de magnésium (1X)
- RIPA buffer (1X)

### Méthode

- Incuber les cellules Aag2 à 28°C / 5% CO2 dans le milieu de culture (i.e., RPMI + 1% PS + 1X NEAA) supplementé avec 10% de FCS décomplementé.
- Lorsque les cellules atteignent 80-90% de confluence, retirer le milieu de culture et le remplacer par du milieu frais sans FBS.
- Incuber à 28°C / 5% CO₂ pendant 48 heures.
- Recueillir le surnageant sans détacher la couche cellulaire.
- Centrifuger le surnageant à 300g pendant 10 minutes à 4°C.
- Recueillir le surnageant.
- Centrifuger le surnageant à 2 000g pendant 10 minutes à 4°C.
- Recueillir le surnageant.
- Centrifuger le surnageant à 10 000g pendant 30 minutes à 4°C.
- Recueillir le surnageant.
- Centrifuger le surnageant à 100 000g pendant 155 minutes à 4°C.
- Jeter le surnageant.
- Laver le culot contenant les VE avec du DPBS 1X glacé.
- Centrifuger le surnageant à 100 000g pendant 155 minutes à 4°C.
- Jeter le surnageant.
- Remettre le culot en suspension dans du DPBS 1X glacé.
- Prélever une fraction du culot remis en suspension pour quantifier la teneur en protéines :
   ∘ Mélanger 1:1 dans le tampon RIPA.
   ∘ Quantifier la teneur en protéines à l'aide de Qubit.
- Conserver la solution de VE à -80°C.

De manière avantageuse, l'utilisation de cette lignée cellulaire permet d'obtenir des quantités de VE adaptées à l'immunisation des animaux.

### Exemple 2 : amplification de l'infection et de la transmission par les lipides contenus dans les VE de moustique

### Matériel

- VE isolées de cellules Aag2
- Cellules HuH7 (clone JTC-39)
- Dulbecco's modified Eagle medium (DMEM, Gibco), supplementé avec 10% de sérum fétal bovin (fetal Bovine Sérum)
- Virus de la Dengue de type 2 (DENV2) souche NGC, virus West Nile (WNV) souche IS98, et virus Zika (ZIKV) souche PF-251013-18
- Souris C57BI6/J, mâles agés de 4-5 semaines (Charles River Laboratories)
- Méthanol:Chlorform:eau exempte de nucléase (2:1:1 volume)
- Méthanol:chloroforme (1:1 volume)
- DMSO (diméthylsulfoxyde)

### Méthode d'extraction des lipides

- Les VE d'Aag2 sont mélangés avec du Méthanol:Chlorforme:eau sans nucléase
- Vortexer et centrifuger le mélange
- Recueillir la phase liquide monophasique
- Sécher le liquide sous azote
- Resuspendre le culot dans du DMSO
- Ajouter la solution de méthanol et de chloroforme
- Mélanger à l'aide d'un vortex et centrifuger le mélange
- Sécher le liquide sous azote
- Remettre le culot en suspension dans le DMSO

### Méthode pour l'infection des cellules avec des lipides contenus dans les VE

- Mettre sur plaque les cellules Huh7
- Infecter avec DENV, ZIKV ou WNV à une multiplicité d'infection (MOI = 0,1)
- Supplémenter l'inoculum avec des extrais de lipides contenus dans les VE
- Les cellules contrôle reçoivent le même volume de DMSO
- Incuber les cellules pendant 72 heures après l'infection
- Prélever les cellules et quantifier les génomes viraux (ARNg) par RT-qPCR

### Méthode d'infection des souris avec des lipides contenus dans les VE

- Dès réception des souris, celles-ci sont placées dans des cages par groupes de 4 à 5 avec de la nourriture et de l'eau à volonté.
- Attendre une semaine pour que les souris s'acclimatent à leur nouvel environnement.
- Anesthésier les souris
- Injecter en intradermique à chaque souris 10µL d'une solution contenant 1000 particles formant unité de virus West Nile, d'extraits de lipides contenus dans les VE et du PBS.
- Des souris témoins ont été injectées avec 10µl de virus West Nile, un volume équivalent de DMSO et de PBS.
- Un deuxième groupe de souris témoins a reçu un volume équivalent de DMSO et de PBS, sans le virus West Nile.
- Les souris sont remises dans leurs cages.
- Les signes cliniques et la survie sont surveillés tous les jours jusqu'à 12 jours après l'injection.

Dans le cadre de l'invention, il a été montré que les lipides contenus dans les VE de moustique amplifient l'infection dans des cellules humaines et la transmission virale dans un modèle souris (Fig. 1A, B et C). Notamment, il a été montré que les lipides de VE augmentent l'infection par plusieurs flavivirus (le virus de la dengue (DENV), le virus du Zika (ZIKV) et le virus de la fièvre du Nil Occidental (WNV)) (Fig. 1A), que les sphyngomyélines de VE augmentent la traduction des protéines virales et que de ce fait, la transmission virale est augmentée.

En outre, il a été montré dans le cadre de l'invention que la supplémentation en lipides de VE augmente l'infection par plusieurs flavivirus, notamment le virus de la dengue, le virus Zika et le virus West Nile, sur plusieurs modèles cellulaires pertinents pour la transmission, comme des cellules primaires de fibroblastes cutanées et cellules immunitaires infectées prioritairement par les virus lors de la piqûre. De plus, l'effet des lipides des VE a été validé avec un modèle murin en montrant que l'injection de lipides avec du virus augmente l'infection de l'animal (Fig 1B et C).

En conclusion, nos résultats ont identifié un facteur d'amplification de la transmission pan-flavivirale.

### Exemple 3 : Immunisation de souris de type sauvage contre les vésicules extracellulaires

Les VE purifiées comme décrit à l'exemple 1 sont utilisées pour immuniser des souris de type sauvage (sans modification génétique) pour étudier la réponse immunitaire dans des conditions où l'immunité est complète.

10µg de VE purifiées et intacte (diluées dans le PBS) en combinaison avec l'adjuvant Alum sont injectés dans des souris de type sauvage deux fois à deux semaines d'intervalle (Fig. 3A).

Des souris contrôles n'ont reçu aucune injection.

Pour évaluer l'effet de l'immunisation contre les VE sur la transmission par piqûre, des moustiques *Aedes aegypti* ont été injectés par inoculation intrathoracique avec le virus du Nil Occidental, afin d'homogénéiser l'inoculum reçu par chaque moustique et donc leur niveau d'infection.

Les souris immunisées contre les VE et les souris contrôles ont été piquées par 3 moustiques infectés, pour lesquels l'infection a été validée. Les symptômes et la virémie ont été quantifiés dans les souris pendant deux semaines après la dernière immunisation. Préalablement, il a été établi que, dans les mêmes conditions sans immunisation, les symptômes apparaissent avant 7 jours post piqûre.

Les résultats montrent que les souris immunisées ont moins de symptômes d'infection (évalués par la sévérité des symptômes ; Fig. 2A) et une meilleur survie (Fig. 2B) par rapport aux souris contrôles.

Ces résultats montrent que l'immunisation des souris a permis d'attenuer la transmission virale et la pathologie liée à l'infection systémique. Ainsi, une vaccination contre les VE de moustique protège contre l'infection par piqûre de flavivirus transmis par les moustiques.

### Matériel

- VE isolées de cellules Aag2 comme décrit dans l'exemple 1
- solution saline tamponnée au phosphate de Dulbecco (DPBS), dépourvue de calcium et de magnésium (1X)
- Alhydrogel^{®} adjuvant 2% (InvivoGen)
- Souris C57BI6/J mouse, male, agées de 4-5-semaines (Charles River Laboratories)

### Méthode

- Dès leur arrivée, les souris sont placées dans des cages par groupes de 4 à 5 et bénéficient d'un enrichissement, de nourriture et d'eau à volonté.
- Aclimatation d'une semaine des souries à leur nouvel environnement.
- Préparation de la solution d'immunisation pour le nombre requis de souris. Pour 1 souris: 50µL d'adjuvant Alhydrogel 2% + 50µL VE (10µg d'équivalent protéines) dilué dans du PBS.
- Injecter à chaque souris, par voie intrapéritonéale, 100 µl de solution d'immunisation.
- Replacement des souris dans leurs cages.
- Après 2 semaines, injecter les souris avec la même solution d'immunisation (booster).
- Replacement des souris dans leurs cages.
- Attendre 2 semaine avant le challenge viral par piqûre de moustique.
- Les souris contrôle ne subissent aucune injection.

### REFERENCES

1. Peleg, J. "Growth of Arboviruses in Primary Tissue Culture of Aedes Aegypti Embryos." American Journal of Tropical Medicine and Hygiene 17, no. 2 (1968): 219-.

## Revendications

1. Vésicule extracellulaire isolée de moustique pour son utilisation comme agent immunogène dans la prévention d'au moins une maladie à transmission vectorielle.

2. Vésicule extracellulaire pour son utilisation selon la revendication 1, chez un vertébré humain ou non humain.

3. Vésicule extracellulaire pour son utilisation selon la revendication 1 ou 2, le moustique étant choisi parmi le genre Aedes, par exemple Aedes *aegypti*, *Aedes albopictus*, ou *Aedes Japonicus*, parmi le genre *Culex,* par exemple *Culex pipiens*, *Culex quinquefasciatus*, *Culex tarsalis*, *Culex molestus* ou *Culex tritaeniorhynchus,* ou parmi le genre *Anopheles,* par exemple *Anopheles gambiae*, *Anopheles coluzii*, ou *Anopheles arabiensis.*

4. Vésicule extracellulaire pour son utilisation selon l'une quelconque des revendications précédentes, ladite vésicule étant isolée à partir d'au moins un liquide biologique sécrété par ledit moustique, ou d'au moins un extrait cellulaire de moustique.

5. Vésicule extracellulaire pour son utilisation selon la revendication 4, ledit liquide biologique étant choisi parmi la salive, l'hémolymphe ou les excrétats, et ledit extrait cellulaire étant choisi parmi une culture de cellules, un extrait de glandes salivaire, un broyat d'oeufs, un broyat d'intestin, un broyat de larves, ou un broyat du corps du moustique, ou un culture *ex vivo* de tissus ou d'organe de moustiques.

6. Vésicule extracellulaire pour son utilisation selon l'une quelconque des revendications précédentes, le virus étant transmis par un vecteur, ledit virus étant au moins un virus choisi parmi les flavivirus.

7. Vésicule extracellulaire pour son utilisation selon l'une quelconque des revendications précédentes, ladite maladie étant au moins une maladie choisie parmi la dengue, le Zika, la fièvre jaune, l'encéphalite japonaise, la fièvre du Nil Occidental, l'encéphalite de Saint Louis, la maladie causée par le virus Tembusu et la maladie causée par le virus Usutu.

8. Composition vaccinale comprenant au moins une vésicule extracellulaire de moustique telle que définie dans la revendication 4 ou 5.

9. Composition vaccinale selon la revendication 8, comprenant en outre au moins une substance choisie parmi les véhicules, les excipients, les adjuvants, les tampons, les conservateurs, les régulateurs immunitaires et les stabilisants.

10. Composition vaccinale selon la revendication 8 ou 9, ladite composition étant sous forme injectable.

11. Procédé de préparation de vésicule extracellulaire de moustique, comprenant les étapes suivantes :
1) culture de cellules de moustique, non infectées ou infectées par un virus,
2) séparation du surnageant de culture,
3) purification des vésicules extracellulaires du surnageant de culture, par exemple par ultracentrifugation.

12. Procédé selon la revendication 11, dans lequel lesdites cellules sont choisies parmi les lignées cellulaires *Aedes aegypti* Aag2, RML-12 et CCL125, *Aedes albopictus* C6/36, C7-10 et U4.4, *Culex quinquefasciatus* Hsu, *Culex tritaeniorhynchus* TRA-171 et *Anopheles gambiae* Mos.55, Sua1B ou 4a-3B.

13. Utilisation d'une vésicule extracellulaire de moustique pour identifier au moins un peptide immunogène capables de réduire, chez un vertébré la transmission d'une maladie à transmission vectorielle lors d'une piqûre du vertébré par le vecteur.

14. Procédé d'identification d'au moins un peptide immunogène capable de bloquer, chez un vertébré, la transmission d'une maladie à transmission vectorielle lors d'une piqûre du vertébré par le vecteur, comprenant une étape d'identification in vitro d'au moins une cible d'anticorps présents dans un échantillon de sérum d'animal immunisé contre des vésicules extracellulaires de moustique.

15. Utilisation selon la revendication 13 ou procédé selon la revendication 14, dans laquelle ledit au moins un peptide immunogène correspond à tout ou partie de la partie externe, ou à tout ou partie de la partie interne, ou à tout ou partie de la partie transmembranaire, d'une protéine transmembranaire de ladite au moins une vésicule extracellulaire de moustique.
